# EUROPEAN PATENT APPLICATION

(11) **EP 4 036 217 A1**
(43) Date of publication of application: **03.08.2022**
(21) Application number: 20869752.4
(22) Date of filing: 22.09.2020
(51) Int. Cl.: C12N 1/12, C12P 23/00, C12R 1/89

(54) **METHOD FOR PRODUCING ASTAXANTHIN BY HETEROTROPHIC CULTURE OF HAEMATOCOCCUS PLUVIALIS**

(30) Priority: 23.09.2019 CN 201910901712
(71) Applicant: Bioalgo (WF) Co., Ltd, Weifang, Shandong 261205 (CN); Bioalgo (CY) Co., Ltd, Changyi Weifang, Shandong 261313 (CN)
(72) Inventor: ZHENG, Yubin, Weifang, Shandong 261205 (CN); LIU, Shuaishuai, Weifang, Shandong 261205 (CN); CAO, Peixin, Weifang, Shandong 261205 (CN); DAI, Yunfa, Weifang, Shandong 261205 (CN); XIE, Shiyi, Weifang, Shandong 261205 (CN)
(74) Representative: Vossius & Partner Patentanwälte Rechtsanwälte mbB
(86) International application number: PCT/CN2020/116777
(87) International publication number: WO 2021/057711

(57) **Abstract**

Provided is a method for producing astaxanthin, comprising: (a) performing heterotrophic cultivation of astaxanthin-producing *Haematococcus pluvialis;* (b) performing heterotrophic cultivation of the *Haematococcus pluvialis* obtained in step (a) under conditions of high acetate concentration and high dissolved oxygen concentration, where the acetate concentration is at least 1800 mg/L and the dissolved oxygen concentration is at least 2.0 mg/L; and (c) collecting algae cells from step (b) and/or harvesting astaxanthin. Also provided is a base medium for culturing *Haematococcus pluvialis,* a propagation feed medium, and an induction feed medium.

## Description

### Technical Field

The present invention relates to the field of production of astaxanthin, in particular to a method of producing astaxanthin by using *Haematococcus pluvialis,* and to a culture medium used in the method.

### Background Art

Astaxanthin, with a chemical name 3,3'-dihydroxy-4,4'-diketo-β-carotene, a molecular formula of C₄₀H₅₂O₄, and a molecular weight of 596.86, is a keto-carotenoid. In the astaxanthin molecule, there are a long conjugated double bond, and a hydroxyl group as well as an unsaturated ketone at the end of the conjugated double bond chain, where the hydroxyl group and the ketone group constitute an α-hydroxy ketone. These structures have relatively active electronic effect, and may provide electrons to free radicals or attract unpaired electrons of free radicals, making them highly reactive with free radicals to scavenge free radicals, thereby playing an anti-oxidant effect. Its anti-oxidant ability is 38 times higher than that of β-carotene, and 500 times higher than that of vitamin E, thereby having effects such as improving immunity, protecting eyes, protecting cardiovascular and cerebrovascular, anti-inflammation, improving joint pain, beauty and sun protection, etc., for which it is widely used in various fields such as health care products, and cosmetics.

Astaxanthin may be obtained by chemical synthesis or by extraction from a natural raw material. The chemically synthesized astaxanthin contains three photoisomers: laevo- (3S-3'S), meso- (3R-3'S), and dextro- (3R-3'R) photoisomers, whose antioxidant activity and biological safety are not as good as fully-laevo-astaxanthin derived from a natural raw material, particularly from an alga. *Haematococcus pluvialis* is a unicellular chlorophyceae capable of accumulating a large amount of astaxanthin to appear red, with a content of up to 1.5-3% of dry cell weight, and it is at present the main source of natural astaxanthin.

*Haematococcus pluvialis* has a life cycle divided into two stages: vegetative cells and chlamydospores. Under a suitable environment and a nutrient-rich condition, *Haematococcus pluvialis* grows rapidly, divides and reproduces, thereby producing a large number of vegetative cells with flagella. When the environmental condition becomes unsuitable, motile cells lose flagella and become immotile vegetative cells. Under stimulus of a continuous adverse environmental condition, such as high light, high salt, nutrient starvation, etc., the vegetative cells no longer divide and reproduce, and fight against the adverse environmental condition by accumulating a large amount of astaxanthin in the cells, resulting in formation of red chlamydospores.

At present, it is generally believed that high light is a prerequisite for obtaining a high level of astaxanthin. Under a high light condition, algae cells produce excessive reactive oxygen species (ROS) through photosynthesis. *Haematococcus pluvialis* may synthesize and accumulate astaxanthin to withstand oxidative damage of reactive oxygen species to algae cells (Li et al. 2008, Li et al. 2010). Based on this research theory, *Haematococcus pluvialis* astaxanthin is typically induced by using a shallow runway pond (with a liquid depth of 5-15 cm) or a pipeline photobioreactor with a short light path (3-5 cm), and then performing outdoor cultivation in an area with less rainfall and high light intensity, such as Yunnan in China and Hawaii in the United States. *Haematococcus pluvialis* cells obtained through large-scale cultivation may generally reach a density of 1-2 g/L, with a content of astaxanthin of 1.5-3.0% (w/w) of the dry weight of algae cells, and a yield of astaxanthin of 15-60 mg/L. However, as density of cells is inversely related to penetration of light, a high yield of astaxanthin may only be obtained by reducing cell density, light path of reactor or supplementing artificial light. However, these measures may lead to problems such as increased volume of culture, increased floor space, increased cost of equipment and operation, and difficulty in scaling-up. In addition, as duration and intensity of natural light vary with seasons and weather conditions, the content of astaxanthin is unstable. In addition, due to reasons such as structure, pipeline design, operating cost of reactors, effective sterilization may not be realized in outdoor large-scale cultivation, resulting in inevitable contamination by invasive species such as protozoa, contaminating algae, fungi, and bacteria during the cultivation, which further aggravates the instability of production and the variability of product quality.

In addition to photosynthetic and autotrophic growth by using carbon dioxide as a carbon source, *Haematococcus pluvialis* may also be heterotrophically cultured by using an organic carbon source under a dark condition (Kobayashi et al. 1992). Compared with photosynthetic autotrophy, heterotrophic culture may be scaled up by using a traditional fermenter, thereby implementing industrial production. However, as mentioned above, high light intensity is a key factor for the current large-scale production of astaxanthin. Although it has been reported in the literatures that a high salinity stimulation may also stimulate *Haematococcus pluvialis* to accumulate astaxanthin under a dark heterotrophic condition, as the yield of astaxanthin is too low (9 mg/L), it has no practical value (Kobayashi et al. 1997). In order to exploit the advantages of heterotrophic culture and overcome the bottleneck problem that is difficult to effectively accumulate astaxanthin under dark conditions, in recent years, it has been reported in the literatures that by adopting a heterotrophic-autotrophic tandem culture mode, where algal cells may be expanded in green vegetative cells through heterotrophic culture under a dark condition, and then the green vegetative cells are induced to accumulate astaxanthin by a traditional photoautotrophic manner, the cell density may be up to 6 g/L, and the yield of astaxanthin may reach up to 114 mg/L (Hata et al. 2001, Zhang et al. 2016, Wan et al. 2015). The invention patent application (PCT/CN2013/084262) discloses a method of producing astaxanthin by using *Haematococcus pluvialis,* comprising obtaining high-density green vegetative cells firstly through dark heterotrophic culture; feeding a culture medium for dilution; and then accumulating astaxanthin through culture under light. The density of cells may be up to 1.92 g/L, and the yield of astaxanthin may be up to 43.3 mg/L. These methods adopt heterotrophic culture in the first step of green expanded culture, which improves the productivity of green vegetative cells to a certain extent, but still adopt a traditional photoautotrophy for induction in the second step, which makes them still traditional culture modes in essence. Due to the limitation of light, both the density of *Haematococcus pluvialis* cells and the yield of astaxanthin are very low.

### Summary of the Invention

Unless otherwise defined, all technical and scientific terms used herein have the same meanings as commonly understood by a person skilled in the art. For example, refer to Singleton et al. , DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989).

In order to solve the above shortcomings and defects of the prior art, the present invention provides a method of producing astaxanthin by culturing *Haematococcus pluvialis,* comprising:
(a) heterotrophically culturing an astaxanthin-producing *Haematococcus pluvialis;*
(b) heterotrophically culturing the *Haematococcus pluvialis* in step (a) under a condition of a high concentration of acetate and a high concentration of dissolved oxygen; and
(c) collecting algae cells and/or harvesting astaxanthin, and optionally separating and purifying astaxanthin.

As used herein, the "heterotrophically culturing/heterotrophic culture" is a culture mode that uses an organic carbon source for growth and reproduction under a dark condition. The method steps of heterotrophic culture for growth and reproduction of algae cells are known in the art, such as Shi et al., Heterotrophic production of biomass and lutein by Chlorella protothecoides on various nitrogen sources (Enzyme and Microbial Technology 2000, 27: 312-318).

As used herein, the "dark condition" refers to a condition where there is no light or light insufficient for autotrophic cultivation of algae. As used herein, the "autotrophic/ autotrophically" is a culture mode that uses an inorganic carbon source such as carbon dioxide, a carbonate and a bicarbonate for growth and reproduction through photosynthesis under a light condition.

The "organic carbon source" that can be used in the present invention refers to an organic carbon source that can be utilized by a target microorganism to be cultured. Those skilled in the art may determine which organic carbon sources can be used for culturing algae cells based on the technical knowledge in the art, for example, including but not limited to, acetic acid, acetates, propionic acid, propionates, butyric acid, butyrates, lactic acid, lactates, fatty acid, fatty acid salts, amino acids, methanol, ethanol, glycerin, citric acid, citrates, pyruvic acid, pyruvates, glucose, fructose, arabinose, lactose, mannose, rhamnose, ribose or waste water, hydrolysate, zymotic fluid containing these organic carbon sources, preferably acetic acid and an acetate. The organic carbon source may be added in an amount determined according to the conventional knowledge in the field and the actual growth condition of algae cells, which are all within the technical ability of those skilled in the art. In one embodiment, the organic carbon source used in step (a) is acetic acid or an acetate.

The astaxanthin-producing *Haematococcus pluvialis* described herein may be any species of *Haematococcus pluvialis* that can produce astaxanthin, for example, including but not limited to: *Haematococcus pluvialis* CCTCC M2018809, *Haematococcus pluvialis* AC136, AC143, AC587, AC588 (Algobank-Caen Microalgal Culture Collection of University of Caen Basse-Normandie, France), *Haematococcus pluvialis* ATCC 30453, ATCC 30402 (American Type Culture Collection, USA), *Haematococcus pluvialis* CS-321 (Australian National Algae Culture Collection, Australia), *Haematococcus pluvialis* G 1002 (Culture Collection of Algae of Charles University, Czech Republic), *Haematococcus pluvialis* ETTL 1958/3, TAKACOVAL 1983/1, PRIBYL 2005/4, PRIBYL 2008/3 (Culture Collection of Autotrophic Organisms, Czech Republic), *Haematococcus pluvialis* CCCryo 188-04, CCCryo 189-04, CCCryo 190-04 (Culture Collection of Cryophilic Algae, Germany), *Haematococcus pluvialis* SCCAP K-0084 (Scandinavian Culture Collection of Algae and Protozoa at the University of Copenhagen, Denmark), *Haematococcus pluvialis* IPPAS H-239 (Culture Collection of Microalgae, Institute of Plant Physiology, Russian Academy of Science, Russia), *Haematococcus pluvialis* NIVA-CHL 9 (Norwegian Institute for Water Research Culture Collection of Algae, Norway), *Haematococcus pluvialis* FWAC 7072, FWAC 7039 (Canadian Center for the Culture of Microorganisms, Canada), *Haematococcus pluvialis* CPCC 93 (Canadian Phycological Culture Centre of University of Waterloo, Canada), *Haematococcus pluvialis* ACOI 816, ACOI 815, ACOI 276, ACOI 255, ACOI 133, ACOI 51 (Coimbra Culture Collection of Algae, Portugal), *Haematococcus pluvialis* CCAP 34/1D, CCAP 34/1F, CCAP 34/6, CCAP 34/7, CCAP34/8, CCAP 34/12, CCAP 34/13, CCAP 34/14 (Culture Collection of Algae and Protozoa of the Centre for Hydrology and Ecology, UK), *Haematococcus pluvialis* NIES-144, NIES-2263, NIES-2264, NIES-2265 (Microbial Culture Collection of National Institute for Environmental Studies, Japan), *Haematococcus pluvialis* SAG 192.80, SAG 44.96, SAG 34-1a, SAG 34-1b, SAG 34-1c (Culture Collection of Algae at University of Göttingen, Germany), *Haematococcus pluvialis* CCAC 0055, CCAC 0125, CCAC 0129, CCAC 2072B (Culture Collection of Algae at the University of Cologne, Germany), *Haematococcus pluvialis* UTEX 2505, UTEX 16, UTEX B 294 (University of Texas Culture Collection of Algae, USA), *Haematococcus pluvialis* CWU-MACC20 (Herbarium of Kharkov University-MicroAlgae Cultures Collection, Ukraine), *Haematococcus pluvialis* TISTR 8647 (Thailand Institute of Scientific and Technological Research Culture Collection, Thailand), *Haematococcus pluvialis* FACHB-712, FACHB-827, FACHB-797, FACHB-955, FACHB-1164 (Freshwater Culture Algae Collection at Institute of Hydrobiology, The Chinese Academy of Sciences, China) 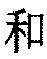 *Haematococcus pluvialis* CCMP 3127 (Provasoli-Guillard National Centre for Marine Algae and Microbiota, USA).

*Haematococcus pluvialis* AQHP0 was deposited at the China Center for Type Culture Collection (CCTCC) (Wuhan University, Wuhan, China, 430072) under accession number of CCTCC M 2018809 on November 21, 2018.

In one embodiment, the heterotrophic culture in step (a) is carried out at a pH of about 6.0-9.0, for example about 6.0-8.0, 7.0-9.0, 7.0-8.5 or 7.5-8.0. In one embodiment, the pH of the step (a) is about 7.0-8.0. In one embodiment, the pH of the step (a) is about 6.5, 7.0, 7.5, 8.0, or 8.5.

In one embodiment, the step (a) is carried out at a culture temperature of about 15-25°C, for example about 20-25°C, particularly about 15°C, 16°C, 17°C, 18°C, 19°C, 20°C, 21°C, 22°C, 23°C, 24°C, or 25°C. In a preferred embodiment, the step (a) is carried out at a culture temperate of about 20°C or 25°C.

In one embodiment, the concentration of the dissolved oxygen in the broth in step (a) is about 0.1-4.0 mg/L, for example, about 0.5-4.0, 1.0-4.0, 1.0-2.5, 1.0-2.0, 1.5-2.5 or 2.0-4.0 mg/L. In one embodiment, the concentration of the dissolved oxygen in the broth in step (a) is about 0.5, 1.0, 1.5, 2.0, 2.5, 3.0 or 3.5 mg/L.

In one embodiment, the concentration of the acetate in the broth in step (a) is about 60-1200 mg/L, for example about 100-1200, 100-1100, 100-1000, 100-900, 300-1200, 300-1100, 300-1000, 300-900, 300-700 mg/L. In one embodiment, the concentration of the acetate in the broth in step (a) is about 60, 70, 80, 90, 100, 200, 300, 400, 500, 600, 700, 800, 900, 1000, 1100, 1200 mg/L.

In one embodiment, the concentration of the nitrate in the broth in step (a) is about 100-2000 mg/L, for example, about 100-1500, 100-1000, 100-900, 100-400, 300-1500, 300-1000, 300-900, 600-900 mg/L. In one embodiment, the concentration of the nitrate in the broth in step (a) is about 2000, 1900, 1800, 1700, 1600, 1500, 1400, 1300, 1200, 1100, 1000, 900, 800, 700, 600, 500,400, 300, 200 or 100 mg/L.

In one embodiment, the concentration of the phosphate ion in the broth in step (a) is about 50-500 mg/L, for example, about 50-400, 50-300, 50-250, 50-100, 100-250, 150-250, 100-150 mg/L. In a preferred embodiment, the concentration of the phosphate ion in the broth in step (a) is about 100-250, 100-150 or 150-250 mg/L.

In one embodiment, after culturing, the step (a) is stopped when the number of the obtained algae cells is at least about 10⁶ cells/mL (e.g. at least about 1.1, 1.2, 1.3, 1.4, 1.5, 2, 3, 4, 5, 6×10⁶ cells/mL, or e.g. 1-10, 2-10, 3-10, 4-10, 5-10, 6-10×10⁶ cells/mL) or the cell density is at least about 1.0 g/L (e.g. at least about 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10.0 g/L, or e.g. 1.0-10, 1.0-8.0, 2.0-8.0, 3.0-8.0 g/L) or at least about 50% (e.g. at least about 60%, 70%, 80%, 90%, 95%, or 100%) of motile vegetative cells become immotile vegetative cells.

In one embodiment, in step (a), *Haematococcus pluvialis* is cultivated in a basal culture medium, and an expansion feeding medium is added during the cultivation. As used herein, the basal culture medium refers to a medium that can be used to cultivate *Haematococcus pluvialis* to allow it grow and reproduce and usually contains a nitrogen source, a phosphorus source, a sulfur source, a magnesium source, a calcium source, a trace element and/or a vitamin. A suitable basal culture medium for a given alga is known in the art. For this, see BG-11, BBM, C medium, MCM and other media.

The "nitrogen source" that can be used in the culture medium according to the invention refers to a nitrogen source that can be utilized by cultivated algae, e.g. including but not limited to nitric acid, a nitrate such as sodium nitrate, nitrite, ammonia water, an ammonium salt (such as ammonium acetate, ammonium chloride, ammonium bicarbonate and ammonium sulfate), urea, an amino acid, a peptone, a yeast extract, a protein powder, a corn steep liquor.

The "phosphorus source" that can be used in the culture medium according to the invention refers to a phosphorus source that can be utilized by cultivated algae, e.g. including but not limited to phosphoric acid or a phosphate such as sodium dihydrogen phosphate, disodium hydrogen phosphate, dipotassium hydrogen phosphate, potassium dihydrogen phosphate, sodium β-glycerophosphate.

The "sulfur source" that can be used in the culture medium according to the invention refers to a sulfur source that can be utilized by cultivated algae, e.g. including but not limited to sulfuric acid or a sulfate such as magnesium sulfate, sodium sulfate.

The "magnesium source" that can be used in the culture medium according to the invention refers to a magnesium source that can be utilized by cultivated algae, e.g. including but not limited to a magnesium salt such as magnesium sulfate, magnesium chloride.

The "calcium source" that can be used in the culture medium according to the invention refers to a calcium source that can be utilized by cultivated algae, e.g. including but not limited to a calcium salt such as calcium chloride, calcium sulfate, calcium nitrate.

The "trace element" that can be used in the culture medium according to the invention refers to a trace element that can be utilized by cultivated algae, for example, including but not limited to one or more of Mn (such as manganese chloride), Zn (such as zinc sulfate), B (such as boric acid), Mo (such as sodium molybdate), Cu (such as copper sulfate), Co (such as cobalt chloride), Fe (such as ferric chloride, ferric sulfate, or the like). The trace element can be added in the culture medium in an amount determined based on conventional knowledge in the field.

The "vitamin" that can be used in the culture medium according to the invention refer to a vitamin that can be utilized by cultivated algae, e.g. including but not limited to vitamin B12, biotin, vitamin B1.

The present invention provides a basal culture medium, comprising: sodium acetate, sodium nitrate, potassium dihydrogen phosphate, dipotassium hydrogen phosphate, magnesium sulfate, calcium chloride, disodium edetate, boric acid, ferric chloride, manganese chloride, zinc sulfate, sodium molybdate, cobalt chloride, copper sulfate, vitamin B12, biotin and vitamin B1. In one embodiment, the basal culture medium according to the invention has a formula of: 0.1-2.0 g/L sodium acetate, 0.1-3.0 g/L sodium nitrate, 50-500 mg/L potassium dihydrogen phosphate, 20-200 mg/L dipotassium hydrogen phosphate, 50-500 mg/L magnesium sulfate, 1-50 mg/L calcium chloride, 0.5-5 mg/L disodium edetate, 0.1-5 mg/L boric acid,100-500 µg/L ferric chloride, 1-100 µg/L manganese chloride, 1-100 µg/L zinc sulfate, 1-100 µg/L sodium molybdate, 0.1-10 µg/L cobalt chloride, 1-100 µg/L copper sulfate, 0-1 µg/L vitamin B12, 0-1 µg/L biotin, and 0-20 µg/L vitamin B1.

After the basal culture medium is prepared according to the above formula, the pH of the culture medium is adjusted to 6.0-9.0, preferably 7.0-8.0 with 1 mol/L diluted sulfuric acid or sodium hydroxide solution. The prepared basal culture medium is added to a cultivation container such as Erlenmeyer flask or fermenter at a loading coefficient of 0.6-0.8, and then autoclaved at 121°C for 15-30 minutes, and then for inoculation and culture after being cooled to 15-20°C.

In one embodiment, the basal culture medium according to the invention comprises sodium acetate of 0.1-2.0 g/L, such as 0.5-1.5 g/L or 0.5-1.1 g/L, preferably 0.8-1.0 g/L. In particular, the basal culture medium comprises about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 g/L of sodium acetate.

In one embodiment, the basal culture medium according to the invention comprises sodium nitrate of 0.1-3.0 g/L, such as 0.3-2.0 g/L or 0.4-1.5 g/L, preferably 0.4-1.0 g/L. In particular, the basal culture medium comprises about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9 or 3.0 g/L of sodium nitrate.

In one embodiment, the basal culture medium according to the invention comprises potassium dihydrogen phosphate of 50-500 mg/L, such as 60-300 mg/L, preferably 60-200 mg/L. In particular, the basal culture medium comprises about 60, 70, 80, 90, 100, 150, 200, 250, 300, 350, 400, 450 or 500 mg/L of potassium dihydrogen phosphate.

In one embodiment, the basal culture medium according to the invention comprises dipotassium hydrogen phosphate of 20-200 mg/L, for example 20-100 mg/L. In particular, the basal culture medium comprises about 20, 30, 40, 50, 60, 70, 80, 90, 100, 150 or 200 mg/L of dipotassium hydrogen phosphate.

In one embodiment, the basal culture medium according to the invention comprises magnesium sulfate of 50-500 mg/L, such as 50-300 mg/L, preferably 50-200 mg/L. In particular, the basal culture medium comprises about 50, 60, 70, 80, 90, 100, 150, 200, 250, 300, 350, 400, 450 or 500 mg/L of magnesium sulfate.

In one embodiment, the basal culture medium according to the invention comprises calcium chloride of 1-50 mg/L, such as 1-30 mg/L, preferably 1-25 mg/L. In particular, the basal culture medium comprises about 5, 10, 15, 20, 25, 30, 35, 40, 45 or 50 mg/L of calcium chloride.

In one embodiment, the basal culture medium according to the invention comprises disodium edetate of 0.5-5 mg/L, such as 1.5-4.0 mg/L, preferably 1.5-3.0 mg/L. In particular, the basal culture medium comprises about 1.0, 1.5, 2.0, 2.5, 3.0, 3.5, 4.0, 4.5 or 5.0 mg/L of disodium edetate.

In one embodiment, the basal culture medium according to the invention comprises boric acid of 0.1-5 mg/L, such as 0.1-4.0 mg/L, preferably 0.1-3.0 mg/L. In particular, the basal culture medium comprises about 0.1, 0.2, 0.3, 0.4, 0.5, 0.6, 0.7, 0.8, 0.9, 1.0, 1.5, 2.0, 2.5, 3.0, 3.5, 4.0, 4.5 or 5.0 mg/L of boric acid.

In one embodiment, the basal culture medium according to the invention comprises ferric chloride of 100-500 µg/L, such as 200-500 µg/L, preferably 200-350 µg/L. In particular, the basal culture medium comprises about 100, 150, 200, 250, 300, 350, 400, 450 or 500 µg/L of ferric chloride.

In one embodiment, the basal culture medium according to the invention comprises manganese chloride of 1-100 µg/L, such as 4-80 µg/L, preferably 1-70 or 4-70 µg/L. In particular, the basal culture medium comprises about 1, 2, 3, 4, 5, 10, 20, 30, 40, 50, 60, 70, 80, 90 or 100 µg/L of manganese chloride.

In one embodiment, the basal culture medium according to the invention comprises zinc sulfate of 1-100 µg/L, such as 10-50 µg/L, preferably 10-35 µg/L. In particular, the basal culture medium comprises about 1, 2, 3, 4, 5, 10, 11, 12, 13, 14, 15, 20, 30, 35, 40, 50, 55, 60, 65, 70, 75, 80, 85, 90, 95 or 100 µg/L of zinc sulfate.

In one embodiment, the basal culture medium according to the invention comprises sodium molybdate of 1-100 µg/L, such as 1-40 µg/L, preferably 1-30 µg/L. In particular, the basal culture medium comprises about 1, 2, 3, 4, 5, 10, 15, 20, 25, 30, 35, 40, 45, 50, 55, 60, 65, 70, 75, 80, 85, 90, 95 or 100 µg/L of sodium molybdate.

In one embodiment, the basal culture medium according to the invention comprises cobalt chloride of 0.1-10 µg/L, such as 1-10 µg/L, preferably 3-7 µg/L. In particular, the basal culture medium comprises about 1, 2, 3, 4, 5, 6, 7, 8, 9 or 10 µg/L of cobalt chloride.

In one embodiment, the basal culture medium according to the invention comprises copper sulphate of 1-100 µg/L, such as 1-70 µg/L, preferably 1-50 µg/L. In particular, the basal culture medium comprises about 1, 5, 10, 20, 30, 40, 50, 60, 70, 80, 90 or 100 µg/L of copper sulphate.

In one embodiment, the basal culture medium according to the invention comprises vitamin B12 of 0-1 µg/L, for example 0-0.1 µg/L. In particular, the basal culture medium comprises about 0.05, 0.1, 0.2,0.3, 0.4, 0.5, 0.6, 0.7, 0.8, 0.9 or 1.0 µg/L of vitamin B12.

In one embodiment, the basal culture medium according to the invention comprises biotin of 0-1 µg/L, for example 0-0.1 µg/L. In particular, the basal culture medium comprises about 0.05, 0.1, 0.2, 0.3, 0.4, 0.5, 0.6,0.7, 0.8,0.9 or 1.0 µg/L of biotin.

In one embodiment, the basal culture medium according to the invention comprises vitamin B1 of 0-20 µg/L, for example 0-10 µg/L. In particular, the basal culture medium comprises about 1, 2, 3, 4, 5, 6, 7, 8, 9, 10, 11, 12, 13, 14, 15, 16, 17, 18, 19 or 20 µg/L of vitamin B1.

As used herein, the expansion feeding medium is a concentrated basal culture medium for supplementing the components consumed during culturing and/or maintaining certain culture condition(s), such as pH, the concentration of certain component such as acetate ion. In one embodiment, the pH of the broth is controlled by feeding the expansion feeding medium, for example, at 7.0-8.5. In one embodiment, the concentration of the acetate in step (a) is controlled to, for example, 300-700 mg/L, by feeding the expansion feeding medium.

The present invention provides an expansion feeding medium, comprising: acetic acid, sodium nitrate, potassium dihydrogen phosphate, magnesium sulfate, disodium edetate, boric acid, ferric chloride, manganese chloride, zinc sulfate, sodium molybdate, cobalt chloride, copper sulfate, vitamin B12, biotin and vitamin B1. In one embodiment, the expansion feeding medium according to the invention has a formula of: 30-600 g/L acetic acid, 2-70 g/L sodium nitrate, 0.2-5.0 g/L potassium dihydrogen phosphate, 0.05-10 g/L magnesium sulfate, 1-40 mg/L disodium edetate, 1-35 mg/L boric acid, 150-4000 µg/L ferric chloride, 30-1000 µg/L manganese chloride, 15-600 µg/L zinc sulfate, 15-500 µg/L sodium molybdate, 3-100 µg/L cobalt chloride and 20-700 µg/L copper sulfate, 0-10 µg/L vitamin B12, 0-10 µg/L biotin, and 0-1000 µg/L vitamin B1.

The expansion feeding medium may be prepared as follows: preparing components other than acetic acid according to the formula, autoclaving at 121°C for 15-30 minutes, and after being cooled to room temperature, adding glacial acetic acid (with a content of acetic acid of over 98%) to a concentration of acetic acid of 30-600 g/L.

In one embodiment, the expansion feeding medium according to the invention comprises acetic acid of 30-600 g/L, preferably 30-60 g/L. In particular, the expansion feeding medium comprises about 30, 40, 50, 100, 120, 150, 200, 250, 300, 350, 400, 500 or 600 g/L of acetic acid.

In one embodiment, the expansion feeding medium according to the invention comprises sodium nitrate of 2-70 g/L, such as 5-50 g/L, preferably 5-10 or 5-7 g/L. In particular, the expansion feeding medium comprises about 2, 3, 4, 5, 6, 7, 8, 9, 10, 15, 20, 25, 30, 35, 40, 45, 50, 55, 60, 65 or 70 g/L of sodium nitrate.

In one embodiment, the expansion feeding medium according to the invention comprises potassium dihydrogen phosphate of 0.2-5.0 g/L, such as 1.0-5.0 g/L, preferably 0.5-1.0 g/L. In particular, the expansion feeding medium comprises about 0.2, 0.3, 0.4, 0.5, 0.6, 0.7, 0.8, 0.9, 1.0, 1.5, 2.0, 2.5, 3.0, 3.5, 4.0, 4.5 or 5.0 g/L of potassium dihydrogen phosphate.

In one embodiment, the expansion feeding medium according to the invention comprises magnesium sulfate of 0.05-10 g/L, such as 0.1-1.0 g/L. In particular, the expansion feeding medium comprises about 0.1, 0.2, 0.3, 0.4, 0.5, 0.6, 0.7, 0.8, 0.9, 1.0, 1.5, 2.0, 3.0, 4.0, 5.0, 6.0, 7.0, 8.0, 9.0 or 10 g/L of magnesium sulfate.

In one embodiment, the expansion feeding medium according to the invention comprises disodium edetate of 1-40 mg/L, such as 1-30 mg/L, preferably 1-25 or 3-24 mg/L. In particular, the expansion feeding medium comprises about 1, 2, 3, 4, 5, 6, 7, 8, 9, 10, 12, 15, 20, 24, 25, 30, 35 or 40 mg/L of disodium edetate.

In one embodiment, the expansion feeding medium according to the invention comprises boric acid of 1-35 mg/L, such as 1-30 mg/L, preferably 1-6 mg/L. In particular, the expansion feeding medium comprises about 1, 2, 3, 4, 5, 6, 7, 8, 9, 10, 12, 15, 20, 30 or 35 mg/L of boric acid.

In one embodiment, the expansion feeding medium according to the invention comprises ferric chloride of 150-4000 µg/L, such as 350-4000 µg/L. In particular, the expansion feeding medium comprises about 150, 200, 250, 300, 350,400, 500, 600, 700, 800, 900, 1000, 1400, 1500, 1750, 1800, 1900, 2000, 2500, 3000, 3500 or 4000 µg/L of ferric chloride.

In one embodiment, the expansion feeding medium according to the invention comprises manganese chloride of 30-1000 µg/L, such as 70-700 µg/L, preferably 50-300 or 70-280 µg/L. In particular, the expansion feeding medium comprises about 30, 40, 50, 60, 70, 80, 90, 100, 140, 150, 200, 250, 280, 300, 350, 400, 500, 600, 700, 800, 900 or 1000 µg/L of manganese chloride.

In one embodiment, the expansion feeding medium according to the invention comprises zinc sulfate of 15-600 µg/L, such as 35-600 µg/L, preferably 30-100 or 35-70 µg/L. In particular, the expansion feeding medium comprises about 15, 20, 25, 30, 35, 40, 50, 60, 70, 80, 90, 100, 140, 150, 175, 200, 250, 300, 350, 400, 500 or 600 µg/L of zinc sulfate.

In one embodiment, the expansion feeding medium according to the invention comprises sodium molybdate of 15-500 µg/L, such as 30-300 µg/L, preferably 30-60 µg/L. In particular, the expansion feeding medium comprises about 15, 20, 30, 40, 50, 60, 70, 80, 90, 100, 120, 150, 200, 300, 350, 400 or 500 µg/L of sodium molybdate.

In one embodiment, the expansion feeding medium according to the invention comprises cobalt chloride of 3-100 µg/L, such as 5-100 µg/L, preferably 7-100 µg/L. In particular, the expansion feeding medium comprises about 3, 4, 5, 6, 7, 8, 9, 10, 11, 12, 13, 14, 15, 20, 30, 35, 40, 50, 60, 70, 80, 90 or 100 µg/L of cobalt chloride.

In one embodiment, the expansion feeding medium according to the invention comprises copper sulphate of 20-700 µg/L, such as 50-500 µg/L, preferably 50-100 µg/L. In particular, the expansion feeding medium comprises about 20, 30, 40, 50, 100, 200, 250, 300, 400, 500, 550, 600 or 700 µg/L of copper sulphate.

The method according to the invention may comprise additional step(s) before the step (a), such as of pre-cultivation, seed activation, which may be carried out by using the basal medium according to the invention.

In one embodiment, the method according to the invention comprises an activation step before the step (a) to obtain *Haematococcus pluvialis* seed solution. The activation step may be carried out by any suitable method known in the art, for example, by standing or shaking flask to cultivate *Haematococcus pluvialis.* In one embodiment, the proportion of green vegetative cells in the seed solution is at least 90%, for example at least 91%, 92%, 93%, 94%, 95%, 96%, 97%, 98%, 99%, or even 100%. In one embodiment, the cell density in the seed solution is at least 40×10⁴ cells/ml, for example at least 50, 55, 60, 70, 80, 90, 100×10⁴ cells/ml. In one embodiment, the seed activation step may be carried out by using the basal culture medium containing Tris (Tris(hydroxymethylaminomethane)), e.g. Tris of 0.1-1 g/L, preferably 0.5-1.0 g/L, more preferably 0.5-0.6 g/L.

In one embodiment, the present method comprises step (a): heterotrophically culturing a seed solution according to the invention to obtain a culture solution, optionally the number of algae cells in the obtained culture solution is at least about 60×10⁴ cells/mL, for example at least about 70, 80, 90, 100, 110, 120, 130, 140, 150, 160, 170, 180, 190, 200, 300, 400, 500, 600, 700, 800, 900×10⁴ cells/mL, such as 100-1000, 150-900, 150-800, 150-700, 150-600×10⁴ cells/mL, and/or the cell density is at least about 1.0 g/L, for example at least about 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10.0 g/L, such as about 1.0-10.0, 1.0-9.0, 1.0-8.0, 1.0-7.0, 1.0-6.0 g/L. In one embodiment, at least 50%, for example, at least about 60%, 70%, 80%, 90%, 95%, or 100% of motile vegetative cells in the obtained culture solution become immotile vegetative cells.

In one embodiment, in step (a), the seed solution inoculates a culture medium at an initial inoculation amount of 1-50, 1-40, 1-30, or 1-20×10⁴ cells/mL, for example, 2, 3, 4, 5, 6, 7, 8, 9, 10, 15, 16, 17, 18, 19, 20, 25, 30, 35, 40, 45×10⁴ cells/mL.

In one embodiment, the activation step is carried out at a pH of 7.0-8.0, preferably 7.5-8.0.

In one embodiment, the activation step is carried out at a culture temperature of 15-25°C, preferably 20-25°C.

Optionally, after obtaining a *Haematococcus pluvialis* seed solution by the activation step, the method may comprise steps of removing the culture medium and/or collecting vegetative cells, and optionally concentrating the vegetative cells. Removing the culture medium, collecting and/or concentrating the vegetative cells may be performed through any suitable method known in the art, such as precipitation (natural sedimentation or centrifugation) or filtration (using a filter or a filter membrane).

In step (b), *Haematococcus pluvialis* is cultured heterotrophically at a condition of high acetate and high dissolved oxygen to produce astaxanthin. *Haematococcus pluvialis* obtained in step (a) may be removed by any suitable means, optionally by means of separation (for example, by centrifugation), and then added to a culture medium with high acetate and high dissolved oxygen. Alternatively, high acetate (such as acetic acid or an acetate such as sodium acetate) may be directly added to *Haematococcus pluvialis* obtained in step (a), and a high concentration of dissolved oxygen is maintained.

In one embodiment, the step (b) comprises adding to *Haematococcus pluvialis* obtained in step (a) acetic acid or an acetate such as sodium acetate, and/or a culture medium containing a high concentration of acetate to increase the concentration of acetate.

In one embodiment, in step (b), the concentration of the acetate in the culture solution is about at least 1800 mg/L, for example, about at least 2000, 2500, 3000, 3500, 4000, 4500, or 5000 mg/L. In one embodiment, in step (b), the concentration of the acetate in the culture solution is about 1800-6000, 2400-4800, 2700-4000, 3000-5000, 3000-4000, 3500-4800 or 4000-6000 mg/L. In a preferred embodiment, in step (b), the concentration of acetate is 3000-4800 mg/L. In a further preferred embodiment, in step (b), the concentration of acetate is 3000-4000 mg/L.

In one embodiment, in step (b), the pH of the culture solution is 6.0-11.0, preferably 7.0-9.0, for example about 6.5, 7.0, 7.5, 8.0, 8.5, 9.0, 9.5, 10.0, 10.5 or 11.0. In one embodiment, the pH in step (b) is 7.5-8.0.

In one embodiment, in step (b), the culture temperature is 20-35°C, for example about 21, 22, 23, 24, 25, 26, 27, 28, 29, 30, 31, 32, 33, 34 or 35 °C. In one embodiment, in step (b), the culture temperature is about 20°C-30°C, for example about 25°C.

In one embodiment, in step (b), the concentration of dissolved oxygen in the culture solution is about at least 2.0 mg/L, for example, about or at least 3.0, 4.0, 5.0, 6.0, 7.0, 8.0, 9.0 mg/L. In one embodiment, in step (b), the concentration of dissolved oxygen in the culture solution is 2.0 mg/L-10 mg/L, for example 2.0-9.0, 2.0-8.0, 2.0-3.0, 4.0-6.0 mg/L. In a preferred embodiment, in step (b), the concentration of dissolved oxygen in the culture solution is 4.0-8.0 mg/L or 5.0-8.0 mg/L.

In a preferred embodiment of the present invention, the concentration of acetate and/or the concentration of dissolved oxygen in step (b) is greater than the concentration of acetate and/or the concentration of dissolved oxygen in step (a).

In one embodiment, in step (b), an induction feeding medium is fed during the heterotrophic culture.

As used herein, the induction feeding medium is a concentrated basal medium or a nutrient-deficient medium, which is used to maintain certain culture condition(s) to induce the production of astaxanthin in *Haematococcus pluvialis* cells.

In one embodiment, the present invention provides an induction feeding medium comprising: 30-600 g/L acetic acid, 0-70 g/L sodium nitrate, 0-5.0 g/L potassium dihydrogen phosphate, 0-1.0 g/L magnesium sulfate, 1-40 mg/L disodium edetate, 1-35 mg/L boric acid, 150-4000 µg/L ferric chloride, 30-1000 µg/L manganese chloride, 15-600 µg/L zinc sulfate, 15-500 µg/L sodium molybdate, 3-100 µg/L cobalt chloride and 20-700 µg/L copper sulfate.

In one embodiment, the concentration of the acetate in step (b) is controlled by feeding the induction feeding medium, preferably to be at least 600 mg/L, preferably 3000-4800 mg/L.

In one embodiment, the induction feeding medium according to the invention comprises acetic acid of 30-600 g/L, preferably 60-600 g/L. In particular, the induction feeding medium comprises about 30, 40, 50, 60, 70, 80, 90, 100, 150, 200, 240, 250, 300, 350, 400, 500 or 600 g/L of acetic acid.

In one embodiment, the induction feeding medium according to the invention comprises sodium nitrate of 0-70 g/L, preferably 0-50 g/L. In particular, the induction feeding medium comprises about 1, 2, 3, 4, 5, 10, 20, 30, 40, 50, 55, 60, 65 or 70 g/L of sodium nitrate.

In one embodiment, the induction feeding medium according to the invention comprises potassium dihydrogen phosphate of 0-5.0 g/L, such as 0.1-5.0 g/L, preferably 0.5-5.0 g/L. In particular, the induction feeding medium comprises about 0.1, 0.2, 0.3, 0.4, 0.5, 0.6, 0.7, 0.8, 0.9, 1.0, 1.5, 2.0, 2.5, 3.0, 3.5, 4.0, 4.5 or 5.0 g/L of potassium dihydrogen phosphate.

In one embodiment, the induction culture medium according to the invention comprises magnesium sulfate of 0-1.0 g/L, such as 0-1.0 g/L, preferably 0.05-1.0 g/L. In particular, the induction feeding medium comprises about 0.05, 0.1, 0.2, 0.3, 0.4, 0.5, 0.6, 0.7, 0.8, 0.9 or 1.0 g/L of magnesium sulfate.

In one embodiment, the induction feeding medium according to the invention comprises disodium edetate of 1-40 mg/L, preferably 1-30 g/L. In particular, the induction feeding medium comprises about 1, 2, 3, 4, 5, 6, 7, 8, 9, 10, 11, 12, 13, 14, 15, 16, 17, 18, 19, 20, 25, 30, 35 or 40 mg/L of disodium edetate.

In one embodiment, the induction feeding medium according to the invention comprises boric acid of 1-35 mg/L, preferably 1-30 mg/L. In particular, the induction feeding medium comprises about 1, 2, 3, 4, 5, 6, 7, 8, 9, 10, 11, 12, 13, 14, 15, 16, 17, 18, 19, 20, 25, 30 or 35 mg/L of boric acid.

In one embodiment, the induction feeding medium according to the invention comprises ferric chloride of 150-4000 µg/L, such as 150-3500 µg/L. In particular, the induction feeding medium comprises about 150, 200, 300, 350, 400, 500, 1000, 1400, 1500, 1750, 2000, 2500, 3000 or 3500 µg/L of ferric chloride.

In one embodiment, the induction feeding medium according to the invention comprises manganese chloride of 30-1000 µg/L, preferably 30-700 µg/L. In particular, the induction feeding medium comprises about 30, 50, 70, 100, 150, 200, 250, 280, 300, 350, 400, 450, 500, 600, 700, 800, 900 or 1000 µg/L of manganese chloride.

In one embodiment, the induction feeding medium according to the invention comprises zinc sulfate of 15-600 µg/L, such as 15-350 µg/L. In particular, the induction feeding medium comprises about 15, 35, 50, 100, 140, 150, 175, 200, 250, 300, 350, 400, 450, 500, 550 or 600 µg/L of zinc sulfate.

In one embodiment, the induction feeding medium according to the invention comprises sodium molybdate of 15-500 µg/L, preferably 15-300 µg/L. In particular, the induction feeding medium comprises about 15, 20, 30, 50, 100, 120, 150, 200, 300, 350, 400, 450 or 500 µg/L of sodium molybdate.

In one embodiment, the induction feeding medium according to the invention comprises cobalt chloride of 3-100 µg/L, such as 3-90 µg/L, preferably 3-70 µg/L. In particular, the induction feeding medium comprises about 3, 4, 5, 6, 7, 8, 9, 10, 20, 30, 35, 40, 50, 60, 70, 75, 80, 85, 90, 95 or 100 µg/L of cobalt chloride.

In one embodiment, the induction feeding medium according to the invention comprises copper sulphate of 20-700 µg/L, preferably 20-500 µg/L. In particular, the induction feeding medium comprises about 20, 30, 40, 50, 60, 70, 80, 90, 100, 200, 250, 300, 350, 400, 450, 500, 550, 600, 650 or 700 µg/L of copper sulphate.

In one embodiment, when the yield of astaxanthin no longer increases or all immotile vegetative cells are converted into spore cells, the step (b) is stopped.

In step (c), collecting algae cells and harvesting astaxanthin as well as purifying astaxanthin may be carried out by any suitable method known in the art. Harvesting algae spore cells and/or astaxanthin in step (c) may be performed by harvesting algae spore cells through any known method (such as sedimentation or centrifugation) and/or disrupting cell wall (through a mechanical, chemical or enzymatic method) and harvesting astaxanthin, optionally separating and/or purifying astaxanthin by any suitable method.

In a preferred embodiment, the present invention provides a method of producing astaxanthin, comprising:
(1) cultivating *Haematococcus pluvialis* to obtain a seed solution, wherein initial pH is 7.0-9.0 and culture temperature is 15-25°C;
(2) heterotrophically culturing the seed solution obtained in step (1), wherein the culturing is performed at a pH of 6.0-9.0 and at a culture temperature of 15-25°C, with a concentration of dissolved oxygen of 0.1 mg/L-4.0 mg/L; and optionally performing step (3) when 50%-100% of motile vegetative cells become immotile vegetative cells;
(3) heterotrophically culturing the culture solution obtained in step (2), wherein the culturing is performed at a pH of 6.0-9.0, at a concentration of acetate of 1800-6000 mg/L, at a culture temperature of 20-35°C and at a concentration of dissolved oxygen of 2.0 mg/L-10.0 mg/L, wherein the concentration of acetate and the concentration of dissolved oxygen in step (3) are respectively greater than the concentration of acetate and the concentration of dissolved oxygen in step (2); and
(4) collecting algae cells and/or harvesting astaxanthin, optionally purifying astaxanthin.

In a further embodiment, in step (1), the proportion of green vegetative cells in the obtained seed solution is not less than 90%.

In a further embodiment, in step (2), the concentration of acetate is 60-1200 mg/L, the concentration of nitrate is 100-2000 mg/L, the concentration of phosphate is 50-500 mg/L, and the proportion of immotile vegetative cells in the obtained culture medium is not less than 50%.

In a further embodiment, in step (1) and/or step (2), *Haematococcus pluvialis* is cultured in a basal medium according to the invention.

In a further embodiment, in step (2), during the culturing, *Haematococcus pluvialis* is added with an expansion feeding medium according to the invention.

In a preferred embodiment, the present invention provides a method of producing astaxanthin, comprising:
(1) seed activation: for example, by cultivation through standing or shaking flask, culturing *Haematococcus pluvialis* to obtain a seed solution, wherein 0.1-1 g/L of Tris (tris(hydroxymethylaminomethane)) is added to the basal culture medium, the initial pH is 7.0-8.0, the culture temperature controlled to be 15-25°C such as 20°C, and the proportion of green vegetative cells in the obtained seed solution is not less than 90%;
(2) heterotrophic expansion: heterotrophically culturing the seed solution obtained in step (1), for example, in the basal medium according to the invention, with an initial inoculum amount of 1-50×10⁴ cells/mL for e.g. 10-20 days, wherein by adding an expansion feeding medium according to the invention, the pH of the broth is maintained at 6.0-9.0, the concentration of acetate is maintained at 60-1200 mg/L, the concentration of nitrate is maintained at 100-2000 mg/L, the concentration of phosphate is maintained at 50-500 mg/L, the culture temperature is controlled to be 15-25°C, and the concentration of dissolved oxygen is controlled to be 0.1-4.0 mg/L during the fermentation process (for example, by adjusting aeration rate at 0.01-0.5 vvm and stirring speed at 30-100 rpm). Optionally, when the number of algae cells reaches 100-1000×10⁴ cells/mL, the cell density is about 1.0-10.0 g/L and/or 50%-100% of motile vegetative cells become immotile vegetative cells, the next step of heterotrophic induction is carried out;
(3) heterotrophic induction: feeding acetic acid or an acetate such as sodium acetate to the culture in step (2), to increase the concentration of acetate in the broth to 1800-6000 mg/L. During the culturing, by feeding the induction feeding medium according to the invention, the pH of the broth is maintained at 6.0-11.0, the concentration of acetate is maintained to 1800-6000 mg/L, and the culture temperature is controlled to be 20-35°C. During fermentation (for example, by adjusting aeration volume at 0.1-2.0 vvm and stirring speed at 60-200 rpm), the concentration of dissolved oxygen is controlled at 2.0-10.0 mg/L, for example, for culturing for 10-30 days. During this process, when the vegetative cells are converted to red thick-walled spore cells, and optionally when the production of astaxanthin no longer increases, the fermentation is stopped;
(4) Collecting algae cells and/or harvesting astaxanthin, optionally purifying astaxanthin.

In a preferred embodiment, the present invention provides a method of producing astaxanthin, comprising:
(1) culturing *Haematococcus pluvialis* in a basal culture medium according to the invention supplemented with Tris (trishydroxymethylaminomethane) (for example 0.1-1 g/L) to obtain a seed solution, wherein initial pH of the culture medium is 7.5-8.0, the culture temperature is controlled at 20-25°C, and the proportion of green vegetative cells in the obtained seed solution is not less than 90%;
(2) culturing heterotrophically the seed solution obtained in step (1) in the basal culture medium according to the invention, wherein initial inoculum amount is 5-20 (for example 5-18)x 10⁴ cells/mL; during the culturing, feeding the expansion feeding medium according to the invention to maintain the pH of the broth at 7.5-8.0, the concentration of acetate at 300-900 mg/L (for example 300-700 mg/L), the concentration of nitrate at 100-900 mg/L (for example 100-400, 300-900 or 600-900 mg/L), the concentration of phosphate ion at 50-250 mg/L (for example 100-250, 100-150 or 150-250 mg/L), the culture temperature is controlled to be 20-25°C, and the concentration of dissolved oxygen is controlled to be 1.0-4.0 mg/L (for example 1.0-2.5 or 1.0-2.0 mg/L); and optionally, when the number of algae cells reaches 400-700 (for example 450 or 600)x 10⁴ cells/mL, the cell density is about 3.0-8.5 g/L (for example 3.4, 7.8 or 8.2 g/L) or at least 90% (for example at least 95% or 100%) of motile vegetative cells are converted into immotile vegetative cells, performing the next step;
(3) feeding a concentrated acetic acid or an acetate such as sodium acetate to the culture in step (2), to increase the concentration of acetate in the broth to 3000-3500 mg/L; and during the culturing, feeding the induction feeding medium according to the invention to maintain the pH of the broth at 7.5-8.0, the concentration of acetate at 3000-5000 mg/L (for example 3000-4000, 3000-4800 or 3500-4800 mg/L), and the culture temperature is controlled to be 20-25°C, and the concentration of dissolved oxygen is controlled to be 4.0-8.0 mg/L (for example 5.0-8.0 mg/L); and optionally when the production of astaxanthin no longer increases:
(4) collecting algae cells and/or harvesting astaxanthin, optionally purifying astaxanthin.

In the present invention, the concentration of dissolved oxygen may be controlled at an appropriate concentration by any suitable means, for example, by adjusting aeration volume and stirring speed, which are all within the knowledge of those skilled in the art.

Unless expressly specified in the context, the word "or" is intended to include "and".

As used herein, "optional" or "optionally" refers to the occurrence or non-occurrence of the event or situation described following the word, and the description includes the occurrence and non-occurrence of the event or situation. For example, an optionally included step refers to the presence or absence of the step.

As used herein, the term "about" refers to a range of values that includes a specific value, which may be reasonably understood by those skilled in the art as similar to the specific value. In embodiments, the term "about" means within the standard error of using a measurement generally accepted in the art. In embodiments, "about" refers to +/- 10% or 5% of the specified value.

As used herein, a defined numerical range should be regarded as disclosing not only the defined numerical range, but also specific numerical values and sub-ranges within the range. For example, pH 6.0-9.0 covers the range of 6.0 to 9.0 and specific values therebetween, as well as smaller ranges therebetween, such as 6.0-8.0, 7.0-8.0, and so on.

Compared with the prior art, the present invention has the following advantages and effects.
(1) In the present invention, *Haematococcus pluvialis* is cultured in a heterotrophic manner, which overcomes the technical bottleneck of the conventional light culture that cannot achieve high-density culture due to the inverse relationship between light penetration and cell density. In an example, upon completion of induction of astaxanthin in *Haematococcus pluvialis,* the density of algal cells reached 37.25 g/L, and the yield of astaxanthin reached 1076.53 mg/L, much higher than the conventional light culture.
(2) No light is required during the entire technological process from seed preparation, heterotrophic expansion cultivation to astaxanthin induction according to the invention, completely getting rid of the dependence in the conventional light cultivation on climate, seasons, and geography, as well as the influence of changes in external environmental conditions on product quality. Heterotrophic culture may be carried out in a conventional fermenter, through precise control of fermentation parameters, so as to achieve all-weather industrialized and stable production.
(3) Compared with the conventional light cultivation, the solution provided by the present invention may greatly reduce the area occupied, water consumption and harvesting cost due to the high cultivation density and no need for light.
(4) The solution provided by the present invention overcomes the contamination problems such as of protozoa, other algae, fungi, and bacteria in the conventional runway ponds or photobioreactor due to the inability of effective sterilization. To autoclave a fermenter at a high temperature effectively avoids contamination by bacteria and ensures product quality.

### Brief Description of the Drawings

FIG. 1 shows a variation curve of the content vs. yield of astaxanthin during heterotrophic induction of *Haematococcus pluvialis* in Example 1.
FIG. 2 shows a variation curve of the content vs. yield of astaxanthin during heterotrophic induction of *Haematococcus pluvialis* in Example 2.
FIG. 3 shows a variation curve of the content vs. yield of astaxanthin during heterotrophic induction of *Haematococcus pluvialis* in Example 3.
FIG. 4 shows a variation curve of the content vs. yield of astaxanthin during heterotrophic induction of *Haematococcus pluvialis* in Example 4.
FIG. 5 shows a variation curve of the content vs. yield of astaxanthin during heterotrophic induction of *Haematococcus pluvialis* in Example 5.
FIG. 6 shows a variation curve of the content vs. yield of astaxanthin during heterotrophic induction of *Haematococcus pluvialis* in Example 6.

The techniques and methods in the present invention are generally carried out according to conventional methods well known in the art and described in the references cited in this specification. The present invention will be further described in combination with the examples. However, it should be understood that these examples are for illustrative purposes only, and not for limiting the present invention.

### Examples

The present invention is further illustrated by the following examples. However, any example or combinations thereof should not be construed as limiting the scope or embodiments of the present invention. The scope of the present invention is defined by the appended claims. In combination with this specification and common knowledge in the field, a person of ordinary skill in the art can clearly understand the scope defined by the claims. Without departing from the spirit and scope of the present invention, those skilled in the art can make any modification or change to the technical solutions of the present invention, and such modifications and changes are also fallen within the scope of the present invention.

### Example 1

A preferred basal culture medium of this example has a formula of: 0.82 g/L sodium acetate, 1.0 g/L sodium nitrate, 152 mg/L potassium dihydrogen phosphate, 64 mg/L dipotassium hydrogen phosphate, 100 mg/L magnesium sulfate, 15 mg/L calcium chloride, 3 mg/L disodium edetate, 3 mg/L boric acid, 350 µg/L ferric chloride, 70 µg/L manganese chloride, 35 µg/L zinc sulfate, 30 µg/L sodium molybdate, 7 µg/L cobalt chloride, and 50 µg/L copper sulfate.

A preferred expansion feeding medium of this example has a formula of: 7.0 g/L sodium nitrate, 1.0 g/L potassium dihydrogen phosphate, 0.3 g/L magnesium sulfate, 6 mg/L disodium edetate, 6 mg/L boric acid, 700 µg/L ferric chloride, 140 µg/L manganese chloride, 70 µg/L zinc sulfate, 60 µg/L sodium molybdate, 14 µg/L cobalt chloride and 100 µg/L copper sulfate. Upon preparation, it was autoclaved at 121°C for 21 minutes, and after cooling to room temperature, added with glacial acetic acid (with a content of acetic acid of over 98%) through an aseptic operation, to provide a concentration of acetic acid of 60 g/L.

A preferred induction feeding medium of this example has a formula of: 50 g/L sodium nitrate, 5.0 g/L potassium dihydrogen phosphate, 1.0 g/L magnesium sulfate, 30 mg/L disodium edetate, 30 mg/L boric acid, 3500 µg/L ferric chloride, 700 µg/L manganese chloride, 350 µg/L zinc sulfate, 300 µg/L sodium molybdate, 70 µg/L cobalt chloride, and 500 µg/L copper sulfate. Upon preparation, it was autoclaved at 121°C for 21 minutes, and after cooling to room temperature, added with glacial acetic acid (with a content of acetic acid of over 98%) through an aseptic operation, to provide a concentration of acetic acid of 600 g/L.

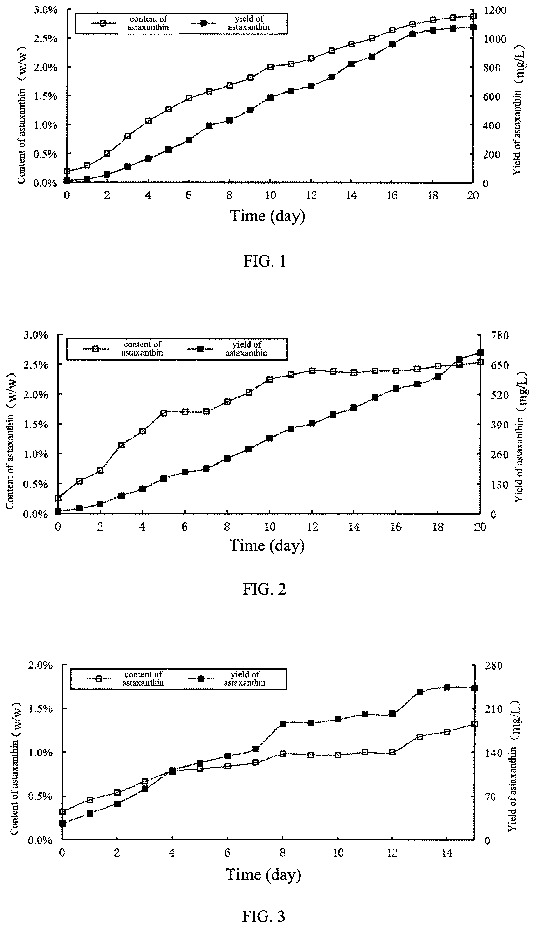

The steps were as follows:
(1) Seed activation: *Haematococcus pluvialis* CCTCC M2018809 (deposited at the China Center for Type Culture Collection (CCTCC)) preserved in a slant medium was inoculated into the sterile basal culture medium with 0.5 g/L Tris (Tris(hydroxymethylaminomethane)) at an initial pH of 7.5, and placed in a 100 mL Erlenmeyer flask at a loading volume of 50 ml for static culture (hand shaking 2 times every day) at a culture temperature of 20°C for 20 days, where 90% of algae cells were green vegetative cells, and the number of algae cells reached 570,000 cells/mL.
(2) Heterotrophic expansion culture: the prepared basal culture medium was added to a 5L fermenter at a loading coefficient of 0.6. The seed solution obtained in step (1) was inoculated into a fermenter at an initial inoculation amount of 180,000 cells/mL for heterotrophic culture, wherein the expansion feeding medium was fed to maintain the pH of the broth at 8.0, the concentration of acetate at 300-900 mg/L, the concentration of nitrate at 600-900 mg/L, the concentration of phosphate ion at 100-150 mg/L, and the culture temperature was controlled at 20°C. During the fermentation, the concentration of dissolved oxygen was controlled at 1.0 mg/L-2.0 mg/L by adjusting aeration volume at 0.1-0.3 vvm and stirring speed at 50-100 rpm. Sampling was conducted every day for observation. After 14 days of cultivation, the density of algal cells reached 7.82 g/L, the number of cells reached 4.5 million cells/mL, and 90% of motile vegetative cells were converted into immotile vegetative cells.
(3) Heterotrophic induction: a concentrated sodium acetate mother solution was added to the fermenter in step (2) through an aseptic operation, to increase the concentration of acetate in the broth to 3000 mg/L, before starting heterotrophic culture. During the culture, the induction feeding medium was fed to maintain the pH of the broth to 8.0, the concentration of acetate at 3000-4000 mg/L, and the culture temperature was controlled at 20°C. During the fermentation, the concentration of dissolved oxygen was controlled at 4.0 mg/L-8.0 mg/L by adjusting aeration volume at 1.0-1.5 vvm and stirring speed at 100-200 rpm. Sampling was conducted every day for observation. After culturing for 20 days, all vegetative cells were converted into red spore cells, and the density of algal cells reached 37.25 g/L. At this time, the content of astaxanthin reached 2.89%, and the yield of astaxanthin reached 1076.53 mg/L (as shown in FIG. 1).

### Example 2

A preferred basal culture medium of this example has a formula of: 0.95 g/L sodium acetate, 0.4 g/L sodium nitrate, 200 mg/L potassium dihydrogen phosphate, 100 mg/L dipotassium hydrogen phosphate, 200 mg/L magnesium sulfate, 25 mg/L calcium chloride, 1.5 mg/L disodium edetate, 1.5 mg/L boric acid, 200 µg/L ferric chloride, 40 µg/L manganese chloride, 20 µg/L zinc sulfate, 20 µg/L sodium molybdate, 3 µg/L cobalt chloride, and 20 µg/L copper sulfate.

A preferred expansion feeding medium of this example has a formula of: 5.0 g/L sodium nitrate, 0.5 g/L potassium dihydrogen phosphate, 0.1 g/L magnesium sulfate, 3 mg/L disodium edetate, 3 mg/L boric acid, 350 µg/L ferric chloride, 70 µg/L manganese chloride, 35 µg/L zinc sulfate, 30 µg/L sodium molybdate, 7 µg/L cobalt chloride and 50 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 60 g/L.

A preferred induction feeding medium of this example has a formula of: 5.0 g/L sodium nitrate, 0.5 g/L potassium dihydrogen phosphate, 0.1 g/L magnesium sulfate, 3 mg/L disodium edetate, 3 mg/L boric acid, 350 µg/L ferric chloride, 70 µg/L manganese chloride, 35 µg/L zinc sulfate, 30 µg/L sodium molybdate, 7 µg/L cobalt chloride, and 50 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 300 g/L.

The steps were as follows:
(1) Seed activation: *Haematococcus pluvialis* CCTCC M2018809 preserved in a slant medium was inoculated into a sterile basal medium with 0.6 g/L Tris at an initial pH of 8.0, and placed in a 100 mL Erlenmeyer flask at a loading volume of 50 ml for static culture (hand shaking 2 times every day) at a culture temperature of 20°C for 20 days, where 95% of algae cells were green vegetative cells, and the number of cells reached 570,000 cells/mL.
(2) Heterotrophic expansion culture: the prepared basal medium was added to a 5L fermenter at a loading coefficient of 0.6. The seed solution obtained in step (1) was inoculated in a fermenter at an initial inoculation amount of 50,000 cells/mL for heterotrophic culture, wherein the expansion feeding medium was fed to maintain the pH of the broth at 8.0, the concentration of acetate at 300-700 mg/L, the concentration of nitrate at 100-400 mg/L, the concentration of phosphate at 150-250 mg/L and the culture temperature was controlled at 20°C. During the fermentation, the concentration of dissolved oxygen was controlled at 1.5 mg/L-2.5 mg/L by adjusting aeration volume at 0.1-0.4 vvm and stirring speed at 60-100 rpm. Sampling was conducted every day for observation. After 15 days of culture, the density of algal cells reached 3.38 g/L, the number of cells reached 4.08 million cells/mL, and 90% of motile vegetative cells were converted to immotile vegetative cells.
(3) Heterotrophic induction: a concentrated sodium acetate mother solution was added to the fermenter in step (2) through an aseptic operation, to increase the concentration of acetate in the broth to 3500 mg/L, before starting heterotrophic culture. During the culture, the induction feeding medium was fed to maintain the pH of the broth at 8.0, the concentration of acetate at 3500-4800 mg/L, and the culture temperature was controlled at 20°C. During the fermentation, the concentration of dissolved oxygen was controlled at 5.0 mg/L-8.0 mg/L by adjusting aeration volume at 1.2-1.8 vvm and stirring speed at 150-200 rpm. Sampling was conducted every day for observation. After 20 days of culture, all vegetative cells were converted to red spore cells, and the density of algal cells reached 27.63 g/L. At this time, the content of astaxanthin reached 2.55%, and the yield of astaxanthin reached 704.57 mg/L (as shown in FIG. 2).

### Example 3

A preferred basal culture medium of this example has a formula of: 0.82 g/L sodium acetate, 0.5 g/L sodium nitrate, 60 mg/L potassium dihydrogen phosphate, 20 mg/L dipotassium hydrogen phosphate, 50 mg/L magnesium sulfate, 1 mg/L calcium chloride, 3 mg/L disodium edetate, 0.1 mg/L boric acid, 350 µg/L ferric chloride, 4 µg/L manganese chloride, 12 µg/L zinc sulfate, 1 µg/L sodium molybdate, 7 µg/L cobalt chloride, 1 µg/L copper sulfate, 0.1µg/L Vitamin B12, 0.1µg/L biotin, and 10 µg/L Vitamin B1.

A preferred expansion feeding medium of this example has a formula of: 6.0 g/L sodium nitrate, 0.7 g/L potassium dihydrogen phosphate, 1.0 g/L magnesium sulfate, 24 mg/L disodium edetate, 1 mg/L boric acid, 4000 µg/L ferric chloride, 280 µg/L manganese chloride, 600 µg/L zinc sulfate, 30 µg/L sodium molybdate, 100 µg/L cobalt chloride and 50 µg/L copper sulfate. Upon preparation, it was autoclaved at 121°C for 21 minutes, and after being cooled to room temperature, added with filter-sterilized Vitamin B12 of 5 µg/L, biotin of 5 µg/L, Vitamin B1 of 500 µg/L, and glacial acetic acid (with a content of acetic acid of over 98%) through an aseptic operation, to provide a concentration of acetic acid of 30 g/L.

A preferred induction feeding medium of this example has a formula of: 0.5 g/L potassium dihydrogen phosphate, 0.05 g/L magnesium sulfate, 1 mg/L disodium edetate, 1 mg/L boric acid, 150 µg/L ferric chloride, 30 µg/L manganese chloride, 15 µg/L zinc sulfate, 15 µg/L sodium molybdate, 3 µg/L cobalt chloride, and 20 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 60 g/L.

The steps were as follows:
(1) Seed activation: *Haematococcus pluvialis* NIES-144 (Microbial Culture Collection of National Institute for Environmental Studies, Japan) preserved in a slant medium was inoculated in a sterile basal medium with 0.5 g/L Tris at an initial pH of 7.5, and placed in a 250 mL Erlenmeyer flask at a loading volume of 50 ml for shake cultivation at 30 rpm and a culture temperature of 25°C for 12 days, where 90% of algae cells were green vegetative cells, and the number of algae cells reached 800,000 cells/mL.
(2) Heterotrophic expansion culture: the prepared basal medium was added to a 5L fermenter at a loading coefficient of 0.6. The seed solution obtained in step (1) was inoculated into a fermenter at an initial inoculation amount of 50,000 cells/mL for heterotrophic culture, wherein the expansion feeding medium was fed to maintain the pH of the broth at 7.5, the concentration of acetate at 300-900 mg/L, the concentration of nitrate at 300-900 mg/L and the concentration of phosphate at 50-100 mg/L, and the culture temperature was controlled at 25°C. During the fermentation, the concentration of dissolved oxygen was controlled at 2.0 mg/L-4.0 mg/L by adjusting aeration volume at 0.1-0.3 vvm and stirring speed at 50-100 rpm. Sampling was conducted every day for observation. After 12 days of culture, the density of algal cells reached 8.15 g/L, the number of cells reached 6.16 million cells/mL, and 95% of motile vegetative cells were converted to immotile vegetative cells.
(3) Heterotrophic induction: a concentrated sodium acetate mother solution was added to the fermenter in step (2) through an aseptic operation, to increase the concentration of acetate in the broth to 3000 mg/L, before starting the heterotrophic culture. During the culture, the induction feeding medium was fed to maintain the pH of the broth at 7.5, the concentration of acetate at 3000-4000 mg/L, and the culture temperature was controlled at 25°C. The temperature was increased to 30°C on Day 10 and to 35°C on Day 12. During the fermentation, the dissolved oxygen was controlled at 4.0 mg/L-8.0 mg/L by adjusting aeration volume at 1.0-1.5 vvm and stirring speed at 100-200 revolutions per minute. Sampling was conducted every day for observation. After culturing for 15 days, all vegetative cells were converted to red spore cells, and the density of algal cells reached 18.35 g/L. At this time, the content of astaxanthin reached 1.33%, and the yield of astaxanthin reached 244.06 mg/L (as shown in FIG. 3).

### Example 4

A preferred basal culture medium of this example has a formula of: 0.50 g/L sodium acetate, 1.5 g/L sodium nitrate, 300 mg/L potassium dihydrogen phosphate, 100 mg/L dipotassium hydrogen phosphate, 300 mg/L magnesium sulfate, 30 mg/L calcium chloride, 4 mg/L disodium edetate, 4 mg/L boric acid, 500 µg/L ferric chloride, 80 µg/L manganese chloride, 50 µg/L zinc sulfate, 40 µg/L sodium molybdate, 10 µg/L cobalt chloride, and 70 µg/L copper sulfate.

A preferred expansion feeding medium of this example has a formula of: 10.0 g/L sodium nitrate, 2.0 g/L potassium dihydrogen phosphate, 0.6 g/L magnesium sulfate, 12 mg/L disodium edetate, 12 mg/L boric acid, 1400 µg/L ferric chloride, 280 µg/L manganese chloride, 140 µg/L zinc sulfate, 120 µg/L sodium molybdate, 30 µg/L cobalt chloride and 200 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 120 g/L.

A preferred induction feeding medium of this example has a formula of: 0.4 g/L magnesium sulfate, 12 mg/L disodium edetate, 12 mg/L boric acid, 1400 µg/L ferric chloride, 280 µg/L manganese chloride, 140 µg/L zinc sulfate, 120 µg/L sodium molybdate, 30 µg/L cobalt chloride, and 200 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 240 g/L.

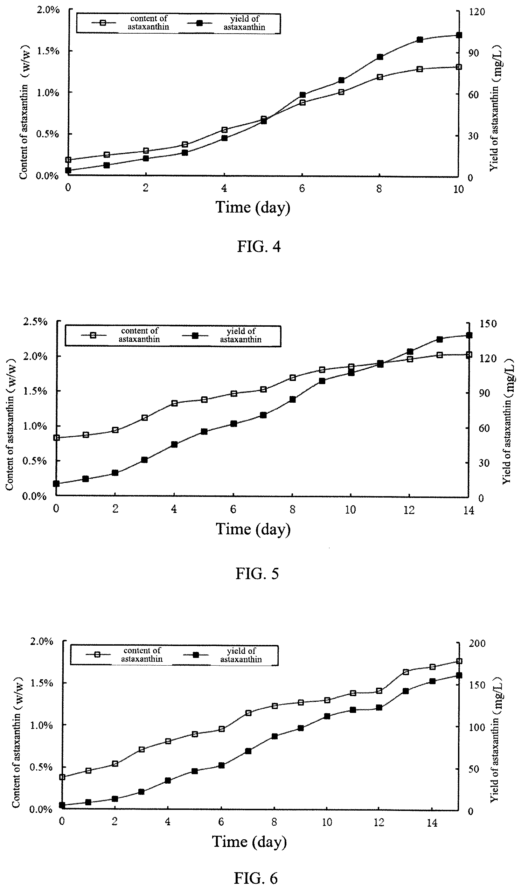

The steps were as follows:
(1) Seed activation: *Haematococcus pluvialis* CCTCC M2018809 preserved in a slant medium was inoculated in a sterile basal medium with 1.0 g/L Tris at an initial pH of 7.0, and placed in a 100 mL Erlenmeyer flask at a loading volume of 50 ml for static culture (hand shaking for 2 times every day) at a culture temperature of 20°C for 20 days, where 92% of algae cells were green vegetative cells, and the number of cells reached 550,000 cells/mL.
(2) Heterotrophic expansion culture: the prepared basal medium was added to a 5L fermenter at a loading coefficient of 0.6. The seed solution obtained in step (1) was inoculated into a fermenter at an initial inoculation amount of 100,000 cells/mL for heterotrophic culture, wherein the expansion feeding medium was fed to maintain the pH of the broth at 8.0, the concentration of acetate at 100-400 mg/L, the concentration of nitrate at 1000-1500 mg/L, and the concentration of phosphate at 200-300 mg/L, and the culture temperature was controlled at 25°C. During the fermentation, the concentration of dissolved oxygen was controlled at 0.1 mg/L-1.0 mg/L by adjusting aeration volume at 0.05-0.15 vvm and stirring speed at 40-60 rpm. Sampling was conducted every day for observation. After 12 days of culture, the density of algal cells reached 1.87 g/L, the number of cells reached was 1.9 million cells/mL, and 70% of motile vegetative cells were converted to immotile vegetative cells.
(3) Heterotrophic induction: a concentrated sodium acetate mother solution was added to the fermenter in step (2) through an aseptic operation, to increase the concentration of acetate in the broth to 2700 mg/L, before starting the heterotrophic culture. During the culture, the induction feeding medium was fed to maintain the pH of the broth at 8.0, the concentration of acetate at 2700-4000 mg/L, and the culture temperature was controlled at 30°C. During the fermentation, the concentration of dissolved oxygen was controlled at 2.0 mg/L-3.0 mg/L by adjusting aeration volume at 0.1-0.5 vvm and stirring speed at 60-100 rpm. Sampling was conducted every day for observation. After culturing for 10 days, all vegetative cells were converted to red spore cells, and the density of algal cells reached 7.74 g/L. At this time, the content of astaxanthin reached 1.32%, and the yield of astaxanthin reached 102.17 mg/L (as shown in FIG. 4).

### Example 5

A preferred basal culture medium of this example has a formula of: 1.10 g/L sodium acetate, 0.7 g/L sodium nitrate, 152 mg/L potassium dihydrogen phosphate, 64 mg/L dipotassium hydrogen phosphate, 100 mg/L magnesium sulfate, 15 mg/L calcium chloride, 3 mg/L disodium edetate, 3 mg/L boric acid, 350 µg/L ferric chloride, 70 µg/L manganese chloride, 35 µg/L zinc sulfate, 30 µg/L sodium molybdate, 7 µg/L cobalt chloride, 50 µg/L copper sulfate, 0.05 µg/L Vitamin B12, 0.05 µg/L biotin, and 5 µg/L Vitamin B1.

A preferred expansion feeding medium of this example has a formula of: 25 g/L sodium nitrate, 2.5 g/L potassium dihydrogen phosphate, 0.5 g/L magnesium sulfate, 15 mg/L disodium edetate, 15 mg/L boric acid, 1750 µg/L ferric chloride, 350 µg/L manganese chloride, 175 µg/L zinc sulfate, 150 µg/L sodium molybdate, 35 µg/L cobalt chloride and 250 µg/L copper sulfate. Upon preparation, it was autoclaved at 121°C for 21 minutes, and after being cooled to room temperature, added with filter-sterilized Vitamin B12 of 2 µg/L, biotin of 2 µg/L, Vitamin B1 of 200 µg/L, and glacial acetic acid (with a content of acetic acid of over 98%) through an aseptic operation, to provide a concentration of acetic acid of 300 g/L.

A preferred induction feeding medium of this example has a formula of: 2.5 g/L potassium dihydrogen phosphate, 0.5 g/L magnesium sulfate, 15 mg/L disodium edetate, 15 mg/L boric acid, 1750 µg/L ferric chloride, 350 µg/L manganese chloride, 175 µg/L zinc sulfate, 150 µg/L sodium molybdate, 35 µg/L cobalt chloride, and 250 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 300 g/L.

The steps were as follows:
(1) Seed activation: *Haematococcus pluvialis* CCTCC M2018809 preserved in a slant medium was inoculated in a sterile basal medium with 0.5 g/L Tris (Tris(hydroxymethylaminomethane)) at an initial pH of 7.5, and placed in a 100 mL Erlenmeyer flask at a loading volume of 50 ml for static culture (hand shaking for 2 times every day) at a culture temperature of 20°C for 20 days, where 98% of algae cells were green vegetative cells, and the number of algae cells reached 600,000 cells/mL.
(2) Heterotrophic expansion culture: the prepared basal medium was added to a 5L fermenter at a loading coefficient of 0.6. The seed solution obtained in step (1) was inoculated into a fermenter at an initial inoculation amount of 20,000 cells/mL for heterotrophic culture, wherein the expansion feeding medium was fed to maintain the pH of the broth at 7.0, the concentration of acetate at 500-900 mg/L, the concentration of nitrate at 400-700 mg/L, and the concentration of phosphate at 100-150 mg/L, and the culture temperature was controlled at 20°C. During the fermentation, the concentration of dissolved oxygen was controlled at 0.5 mg/L-1.5 mg/L by adjusting aeration volume at 0.1-0.2 vvm and stirring speed at 50-70 rpm. Sampling was conducted every day for observation. After 10 days of culture, the density of algal cells reached 1.26 g/L, the number of cells reached was 670,000 cells/mL, and 60% of motile vegetative cells were converted to immotile vegetative cells.
(3) Heterotrophic induction: a concentrated sodium acetate mother solution was added to the fermenter in step (2) through an aseptic operation, to increase the concentration of acetate in the broth to 4800 mg/L, before starting the heterotrophic culture. During the culture, the induction feeding medium was fed to maintain the pH of the broth at 9.0, the concentration of acetate at 4000-6000 mg/L, and the culture temperature was controlled at 30°C. During the fermentation, the concentration of dissolved oxygen was controlled at 4.0 mg/L-8.0 mg/L by adjusting aeration volume at 1.0-1.5 vvm and stirring speed at 100-200 rpm. Sampling was conducted every day for observation. After culturing for 14 days, all vegetative cells were converted to red spore cells, and the density of algal cells reached 6.80 g/L. At this time, the content of astaxanthin reached 2.05%, and the yield of astaxanthin reached 139.40 mg/L (as shown in FIG. 5).

### Example 6

A preferred basal culture medium of this example has a formula of: 0.66 g/L sodium acetate, 0.8 g/L sodium nitrate, 100 mg/L potassium dihydrogen phosphate, 40 mg/L dipotassium hydrogen phosphate, 150 mg/L magnesium sulfate, 20 mg/L calcium chloride, 2 mg/L disodium edetate, 2 mg/L boric acid, 300 µg/L ferric chloride, 50 µg/L manganese chloride, 30 µg/L zinc sulfate, 25 µg/L sodium molybdate, 5 µg/L cobalt chloride, and 30 µg/L copper sulfate.

A preferred expansion feeding medium of this example has a formula of: 50 g/L sodium nitrate, 5 g/L potassium dihydrogen phosphate, 1 g/L magnesium sulfate, 30 mg/L disodium edetate, 30 mg/L boric acid, 3500 µg/L ferric chloride, 700 µg/L manganese chloride, 350 µg/L zinc sulfate, 300 µg/L sodium molybdate, 70 µg/L cobalt chloride and 500 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 600 g/L.

A preferred induction feeding medium of this example has a formula of: 0.1 g/L potassium dihydrogen phosphate, 1 mg/L disodium edetate, 1 mg/L boric acid, 150 µg/L ferric chloride, 30 µg/L manganese chloride, 15 µg/L zinc sulfate, 15 µg/L sodium molybdate, 3 µg/L cobalt chloride, and 20 µg/L copper sulfate. Upon preparation, it was autoclaved at 121°C for 21 minutes, and after being cooled to room temperature, added with glacial acetic acid (with a content of acetic acid of over 98%) through an aseptic operation, to provide a concentration of acetic acid of 60 g/L.

The steps are as follows:
(1) Seed activation: *Haematococcus pluvialis* CCTCC M2018809 preserved in a slant medium was inoculated in a sterile basal medium with 0.7 g/L Tris at an initial pH of 7.8, and placed in a 100 mL Erlenmeyer flask at a loading volume of 50 ml for static culture (hand shaking for 2 times every day) at a culture temperature of 20°C for 20 days, where 95% of algae cells were green vegetative cells, and the number of cells reached 490,000 cells/mL.
(2) Heterotrophic expansion culture: the prepared basal medium was added to a 5L fermenter at a loading coefficient of 0.6. The seed solution obtained in step (1) was inoculated into a fermenter at an initial inoculation amount of 60,000 cells/mL for heterotrophic culture, wherein the expansion feeding medium was fed to maintain the pH of the broth at 8.0, the concentration of acetate at 200-600 mg/L, the concentration of nitrate at 300-800 mg/L, and the concentration of phosphate at 80-120 mg/L, and the culture temperature was controlled at 20°C. During the fermentation, the concentration of dissolved oxygen was controlled at 0.5 mg/L-1.5 mg/L by adjusting aeration volume at 0.1-0.2 vvm and stirring speed at 50-70 rpm. Sampling was conducted every day for observation. After 8 days of culture, the density of algal cells reached 1.19 g/L, the number of cells reached was 1.5 million cells/mL, and 50% of motile vegetative cells were converted to immotile vegetative cells.
(3) Heterotrophic induction: During the culture, the induction feeding medium was fed to maintain the pH of the broth at 8.0, the concentration of acetate at 600-900 mg/L, and the culture temperature was controlled at 25°C. During the fermentation, the concentration of dissolved oxygen was controlled at 4.0 mg/L-6.0 mg/L by adjusting aeration volume at 0.5-1.0 vvm and stirring speed at 150-200 rpm. Sampling was conducted every day for observation. After culturing for 15 days, all vegetative cells were converted to red spore cells, and the density of algal cells reached 9.07 g/L. At this time, the content of astaxanthin reached 1.78%, and the yield of astaxanthin reached 161.45 mg/L (as shown in FIG. 6).

### References

Li, Y., Sommerfeld, M., Chen, F. & Hu, Q. 2008. Consumption of oxygen by astaxanthin biosynthesis: a protective mechanism against oxidative stress in Haematococcus pluvialis (Chlorophyceae). J. Plant Physiol. 165:1783- 1797.
Li, Y., Sommerfeld, M., Chen, F. & Hu, Q. 2010. Effect of photon flux densities on regulation of carotenogenesis and cell viability of Haematococcus pluvialis (Chlorophyceae). J. Appl. Phycol. 22:253-263
Hata N, Ogbonna JC, Hasegawa Y, Taroda H, Tanaka H. 2001. Production of astaxanthin by Haematococcus pluvialis in a sequential heterotrophic-photoautotrophic culture. J Appl Phycol 13(5):395-402.
Wan, M., Zhang, Z., Wang, J., Huang, J., Fan, J., Yu, A., Wang, W., Li, Y.. 2015. Sequential Heterotrophy-Dilution-Photoinduction Cultivation of Haematococcus pluvialis for efficient production of astaxanthin. Bioresour Technol 198:557-563.
Zhang, Z., Wang B., Hu Q., Sommerfeld M., Li Y., Han D. 2016. A New Paradigm for Producing Astaxanthin From the Unicellular Green Alga Haematococcus pluvialis. Biotechnology and Bioengineering 113(10): 2088-2099.
Li, Y., Zhang, Z, Fan, J., Wan, M., Hou, D., Zhang, J., Huang, J., Liang, S., Wang, J., Chen, J., Wang, W. 2013. Method Using micro-algae for high-efficiency production of astaxanthin. PCT/CN201-V84262.
Kobayashi, M., Kakizono, T., Yamaguchi, K., Nishio, N., Nagai, S. 1992. Growth and astaxanthin formation of Haematococcus pluvialis in heterotrophic and mixotrophic conditions. J Ferment Bioeng 74(1):17-20.
Kobayashi, M., Kurimura, Y., Tsuji, Y. 1997. Light-independent, astaxanthin production by the green microalga Haematococcus pluvialis under salt stress. Biotechnol Lett 19(6):507-509.
Shi, X., Zhang, X., Chen, F. 2000. Heterotrophic production of biomass and lutein by Chlorella protothecoides on various nitrogen sources. Enzyme and Microbial Technology 27: 312-318.

## Claims

1. A method of producing astaxanthin, comprising:
(a) heterotrophically culturing an astaxanthin-producing *Haematococcus pluvialis,* optionally until at least about 50% of algal cells are immotile vegetative cells, and/or optionally the number of algal cells after culture is at least about 10⁶ cells/mL, preferably about 2×10⁶ cells/mL, and/or optionally the density of cells is at least about 1.0 g/L, preferably at least about 2.0 g/L;
(b) heterotrophically culturing the *Haematococcus pluvialis* cells obtained in step (a) under a condition of increased concentrations of acetate and dissolved oxygen, optionally until the yield of astaxanthin no longer increases, wherein the concentration of acetate is at least about 1800 mg/L, and the concentration of dissolved oxygen is at least about 2 mg/L; and
(c) collecting the *Haematococcus pluvialis* cells and/or harvesting astaxanthin in step (b), optionally purifying astaxanthin.

2. The method of claim 1, wherein in step (a):
- the pH of the culture solution is about 6.0-9.0, preferably 7.0-8.0; and/or
- the culture temperature is about 15-25°C, preferably 20-25°C; and/or
- the concentration of dissolved oxygen in the culture solution is about 0.1-4.0 mg/L, and is lower than the concentration of dissolved oxygen in step (b); and/or
- the concentration of acetate is about 60-1200 mg/L, preferably about 100-900 mg/L, more preferably about 300-900 mg/L; and/or
- the concentration of nitrate is about 100-2000 mg/L, preferably about 100-1500 mg/L, more preferably about 100-900 mg/L; and/or
- the concentration of phosphate is about 50-500 mg/L, preferably about 50-300 mg/L, more preferably about 50-250 mg/L; and/or
- the initial density of the *Haematococcus pluvialis* cells is at least about 10⁴, preferably 5×10⁴ cells/mL.

3. The method of claim 1 or 2, wherein in step (b):
- the pH of the culture solution is about 6.0-11.0, preferably 7.0-9.0; and/or
- the culture temperature is about 20-35°C, preferably 20-25°C; and/or
- the concentration of acetate is about 1800-6000 mg/L, preferably 3000-5000 mg/L;
and/or
- the concentration of dissolved oxygen is about 2-10 mg/L, preferably 4-8 mg/L.

4. The method of any one of claims 1 to 3, wherein in step (a), the *Haematococcus pluvialis* is cultured in a basal culture medium, and an expansion feeding medium is fed during the culture process,
wherein the basal culture medium comprises: 0.1-2.0 g/L sodium acetate, 0.1-3.0 g/L sodium nitrate, 50-500 mg/L potassium dihydrogen phosphate, 20-200 mg/L dipotassium hydrogen phosphate, 50-500 mg/L magnesium sulfate, 1-50 mg/L calcium chloride, 0.5-5 mg/L disodium edetate, 0.1-5 mg/L boric acid, 100-500 µg/L ferric chloride, 1-100 µg/L manganese chloride, 1-100 µg/L zinc sulfate, 1-100 µg/L sodium molybdate, 0.1-10 µg/L cobalt chloride, 1-100 µg/L copper sulfate, 0-1 µg/L vitamin B12, 0-1µg/L biotin, and 0-20 µg/L vitamin B1,
wherein the expansion feeding medium comprises: 30-600 g/L acetic acid, 2-70 g/L sodium nitrate, 0.2-5.0 g/L potassium dihydrogen phosphate, 0.05-10 g/L magnesium sulfate, 1-40 mg/L disodium edetate, 1-35 mg/L boric acid, 150-4000 µg/L ferric chloride, 30-1000 µg/L manganese chloride, 15-600 µg/L zinc sulfate, 15-500 µg/L sodium molybdate, 3-100 µg/L cobalt chloride, 20-700 µg/L copper sulfate, 0-10 µg /L vitamin B12, 0-10 µg /L biotin, and 0-1000 µg /L vitamin B1.

5. The method of any one of claims 1 to 4, wherein in step (b), a high concentration of acetate is maintained by adding acetic acid or an acetate such as sodium acetate and/or by feeding an induction feeding medium, wherein the induction feeding medium comprises: 30-600 g/L acetic acid, 0-70 g/L sodium nitrate, 0-5.0 g/L potassium dihydrogen phosphate, 0-1.0 g/L magnesium sulfate, 1-40 mg/L disodium edetate, 1-35 mg/L boric acid, 150-4000 µg/L ferric chloride, 30-1000 µg/L manganese chloride, 15-600 µg/L zinc sulfate, 15-500 µg/L sodium molybdate, 3-100 µg/L cobalt chloride and 20-700 µg/L copper sulfate.

6. The method of any one of claims 1 to 5, comprising an activation step before the step (a), which comprises: obtaining a *Haematococcus pluvialis* seed solution, optionally the proportion of green vegetative cells in the seed solution is at least about 90%, and/or optionally the density of cells is at least about 50×10⁴ cells/mL.

7. The method of claim 6, wherein, in the activation step:
- the pH is about 6.0-9.0, preferably 7.0-8.0; and/or
- the culture temperature is about 15-25°C, preferably about 20-25°C.

8. The method of claim 6 or 7, wherein the step (a) comprises heterotrophically culturing the seed solution to obtain a culture solution, wherein at least about 50% of motile vegetative cells in the obtained culture solution are converted to immotile vegetative cells; optionally the initial inoculation amount of the seed solution is about 1-50×10⁴ cells/mL; optionally the number of algal cells in the obtained culture solution is at least about 1, 2, 3, 4, 5 or 6×10⁶ cells/mL, preferably about 1-10×10⁶ cells/mL, and/or optionally the density of algal cells is at least about 2.0 g/L, preferably about 3.0-10.0 g/L.

9. The method of any one of claims 6-8, wherein the activation step comprises the step of culturing the *Haematococcus pluvialis* in a culture medium comprising Tris (tris(hydroxymethylaminomethane)), preferably at a concentration of about 0.1-1.0 g/L, more preferably about 0.5-0.7 g/L, to obtain the seed solution.

10. The method of any one of claims 6-9, wherein in the activation step, the *Haematococcus pluvialis* is cultured in a basal culture medium, wherein the basal culture medium comprises: 0.1-2.0 g/L sodium acetate, 0.1-3.0 g/L sodium nitrate, 50-500 mg/L potassium dihydrogen phosphate, 20-200 mg/L dipotassium hydrogen phosphate, 50-500 mg/L magnesium sulfate, 1-50 mg/L calcium chloride, 0.5-5 mg/L disodium edetate, 0.1-5 mg/L boric acid, 100-500 µg/L ferric chloride, 1-100 µg/L manganese chloride, 1-100 µg/L zinc sulfate, 1-100 µg/L sodium molybdate, 0.1-10 µg/L cobalt chloride, 1-100 µg/L copper sulfate, 0-1 µg/L vitamin B12, 0-1µg/L biotin, and 0-20 µg/L vitamin B1.

11. The method of any one of claims 1-10, wherein the *Haematococcus pluvialis* is selected from the group consisting of *Haematococcus pluvialis* CCTCC M2018809, AC136, AC143, AC587, AC588, ATCC 30453, ATCC 30402, CS-321, G 1002, ETTL 1958/3, TAKACOVAL 1983/1, PRIBYL 2005/4, PRIBYL 2008/3, CCCryo 188-04, CCCryo 189-04, CCCryo 190-04, SCCAP K-0084, IPPAS H-239, NIVA-CHL 9, FWAC 7072, FWAC 7039, CPCC 93, ACOI 816, ACOI 815, ACOI 276, ACOI 255, ACOI 133, ACOI 51, CCAP 34/1D, CCAP 34/1F, CCAP 34/6, CCAP 34/7, CCAP34/8, CCAP 34/12, CCAP 34/13, CCAP 34/14, NIES-144, NIES-2263, NIES-2264, NIES-2265, SAG 192.80, SAG 44.96, SAG 34-1a, SAG 34-1b, SAG 34-1c, CCAC 0055, CCAC 0125, CCAC 0129, CCAC 2072B, UTEX 2505, UTEX 16, UTEX B 294, CWU-MACC20, TISTR 8647, FACHB-712, FACHB-827, FACHB-797, FACHB-955, FACHB-1164 and CCMP 3127.

12. The method of any one of claims 1-11, comprising:
(1) culturing the *Haematococcus pluvialis* in a basal culture medium containing 0.5-0.6 g/L Tris (trishydroxymethylaminomethane) to obtain a seed solution, wherein the pH of the culture medium in the beginning of culture is 7.5-8.0 and the culture temperature is about 20-25°C, and the proportion of green vegetative cells in the obtained seed solution is at least about 90%;
(2) heterotrophically culturing the seed solution in step (1) in a basal culture medium to obtain a culture solution, wherein during the culturing, the pH is maintained at about 7.5-8.0, the culture temperature is maintained at about 20-25°C, the concentration of dissolved oxygen in the culture solution is maintained at about 1.0-4.0 mg/L, the concentration of acetate is maintained at about 300-900 mg/L, the concentration of nitrate is maintained at about 100-900 mg/L, and the concentration of phosphate is maintained at about 50-250 mg/L, and an expansion feeding medium was fed during the culturing; optionally performing step (3) when at least about 90% of motile vegetative cells are converted to immotile vegetative cells and/or optionally the density of cells in the obtained culture solution is at least 4×10⁶ cells/mL;
(3) adding acetic acid or an acetate such as sodium acetate to the culture solution in step (2) to provide the concentration of acetate of at least about 3000 mg/L and adding an induction feeding medium during the culturing, wherein during the culturing, the pH is maintained at about 7.5-8.0, the concentration of acetate is maintained at least about 3000-5000 mg/L, the culture temperature is maintained at about 20-35°C, preferably about 20-25°C, and the concentration of dissolved oxygen in the culture solution is maintained at about at least 4.0 mg/L, such as about 4.0-8.0 mg/L; and
(4) collecting algae cells and/or harvesting astaxanthin obtained in step (3), optionally purifying astaxanthin;
wherein the basal culture medium comprises: 0.8-1.0 g/L sodium acetate, 0.5-1.0 g/L sodium nitrate, 60-200 mg/L potassium dihydrogen phosphate, 20-100 mg/L dipotassium hydrogen phosphate, 50-200 mg/L magnesium sulfate, 1-25 mg/L calcium chloride, 1.5-3 mg/L disodium edetate, 0.1-3 mg/L boric acid, 200-350 µg/L ferric chloride, 4-70 µg/L manganese chloride, 10-35 or 12-35 µg/L zinc sulfate, 1-30 µg/L sodium molybdate, 3-7 µg/L cobalt chloride, 1-50 µg/L copper sulfate, 0-0.1 µg/L vitamin B12, 0-0.1 µg/L biotin, and 0-10 µg/L vitamin B1;
wherein the expansion feeding medium comprises: 30-60 g/L acetic acid, 5.0-7.0 g/L sodium nitrate, 0.5-1.0 g/L potassium dihydrogen phosphate, 0.1-1.0 g/L magnesium sulfate, 3-24 mg/L disodium edetate, 1-6 mg/L boric acid, 350-4000 µg/L ferric chloride, 50-300 or 70-280 µg/L manganese chloride, 35-600 µg/L zinc sulfate, 30-60 µg/L sodium molybdate, 5-100 or 7-100 µg/L cobalt chloride and 50-100 µg/L copper sulfate;
wherein the induction feeding medium comprises: 60-600 g/L acetic acid, 0-50 g/L sodium nitrate, 0.5-5.0 g/L potassium dihydrogen phosphate, 0.05-1.0 g/L magnesium sulfate, 1-30 mg/L disodium edetate, 1-30 mg/L boric acid, 150-3500 µg/L ferric chloride, 30-700 µg/L manganese chloride, 15-350 µg/L zinc sulfate, 15-300 µg/L sodium molybdate, 3-70 µg/L cobalt chloride and 20-500 µg/L copper sulfate.

13. A basal culture medium for culturing *Haematococcus pluvialis,* comprising: 0.1-2.0 g/L sodium acetate, 0.1-3.0 g/L sodium nitrate, 50-500 mg/L potassium dihydrogen phosphate, 20-200 mg/L dipotassium hydrogen phosphate, 50-500 mg/L magnesium sulfate, 1-50 mg/L calcium chloride, 0.5-5 mg/L disodium edetate, 0.1-5 mg/L boric acid, 100-500 µg/L ferric chloride, 1-100 µg/L manganese chloride, 1-100 µg/L zinc sulfate, 1-100 µg/L sodium molybdate, 0.1-10 µg/L cobalt chloride, 1-100 µg/L copper sulfate, 0-1 µg/L vitamin B12, 0-1µg/L biotin, and 0-20 jxg/L vitamin B1.

14. An expansion feeding medium for culturing *Haematococcus pluvialis,* comprising: 30-600 g/L acetic acid, 2-70 g/L sodium nitrate, 0.2-5.0 g/L potassium dihydrogen phosphate, 0.05-10 g/L magnesium sulfate, 1-40 mg/L disodium edetate, 1-35 mg/L boric acid, 150-4000 µg/L ferric chloride, 30-1000 µg/L manganese chloride, 15-600 µg/L zinc sulfate, 15-500 µg/L sodium molybdate, 3-100 µg/L cobalt chloride and 20-700 µg/L copper sulfate, 0-10 µg/L vitamin B12, 0-10 µg/L biotin, and 0-1000 µg/L vitamin B1.

15. An induction feeding medium for inducing *Haematococcus pluvialis* to produce astaxanthin, comprising: 30-600 g/L acetic acid, 0-70 g/L sodium nitrate, 0-5.0 g/L potassium dihydrogen phosphate, 0-1.0 g/L magnesium sulfate, 1-40 mg/L disodium edetate, 1-35 mg/L boric acid, 150-4000 µg/L ferric chloride, 30-1000 µg/L manganese chloride, 15-600 µg/L zinc sulfate, 15-500 µg/L sodium molybdate, 3-100 µg/L cobalt chloride and 20-700 µg/L copper sulfate.
